# EUROPÄISCHE PATENTSCHRIFT

(11) **EP 2 746 383 B1**
(45) Veröffentlichungstag und Bekanntmachung des Hinweises auf die Patenterteilung: **13.04.2016**
(21) Anmeldenummer: 13005962.9
(22) Anmeldetag: 20.12.2013
(51) Int. Cl.: C12M 1/107, C12M 1/00, C02F 1/02, C02F 11/18, D21B 1/00

(54) **Vorrichtung und Verfahren zur Behandlung von Biomasse**
Method and device for processing biomass
Dispositif et procédé destinés au traitement de biomasse

(30) Priorität: 20.12.2012 DE 102012025027
(43) Veröffentlichungstag der Anmeldung: 25.06.2014
(73) Patentinhaber: Reiflock Abwassertechnik GmbH, 76532 Baden-Baden (DE)
(72) Erfinder: Wurz, Dieter, 76530 Baden-Baden (DE)
(74) Vertreter: Petersen, Frank

(56) Entgegenhaltungen:
- EP-A1- 2 338 971
- WO-A1-00/02653
- WO-A1-01/39604
- WO-A2-2010/049815
- DE-A1-102008 046 615
- US-A- 5 622 655
- US-A1- 2009 137 015
- US-A1- 2010 129 888

## Beschreibung

Die Erfindung betrifft ein Verfahren zur Behandlung von Bioabfällen in einer Biogasanlage, die einen Vorlagebehälter für die Vorbereitung eines Gemisches aus Bioabfällen aufweist und eine Mehrstoff- und Mehrphasendüse sowie eine entsprechende Mehrstoff- und Mehrphasendüse für die Behandlung von Biomasse für eine Biogasanlage, mit einem Zentralrohr, das mit einer Düsenmündung im Bereich eines Mischraumes endet, und mit einem Spalt, der die Mündung des Zentralrohres ringförmig umschließt.

Seit mehreren Jahren kommen verstärkt Biogasanlagen für die Energieerzeugung zum Einsatz. In diesen Anlagen wird aus organischem Material unterschiedlichster Herkunft sogenanntes Biogas erzeugt. Beispiele für derartige Anlagen finden sich in der US 5 622 655 A, der US 2010/129888 A1 oder der WO 2010/049815 A2.

Die spezifischen Eigenschaften der eingesetzten Biomasse erfordern spezielle, an diese angepasste Verfahren. Dabei geht es nicht nur um die Frage, ob bestimmte Formen der Biomasse überhaupt in derartigen Biogasanlagen verarbeitet werden können, vielmehr ist anzustreben, bei einer gegebenen Anlagengröße und bei einem entsprechenden Investitionsvolumen einen möglichst großen Biogasoutput zu erzielen und den nicht in Biogas umzusetzenden organischen Gehalt der Reststoffe möglichst weitgehend zu reduzieren.

Bei den Bemühungen um ein Erreichen des vorstehend umrissenen Ziels bereiten insbesondere Holz und holzähnliche Stoffe (Getreidestroh, Maissilage, usw.) große Probleme. Erwähnt seien hier die schon einige Jahrzehnte zurückliegenden Bemühungen in holzreichen Ländern, spezielle Rassen von Kühen zu züchten, die in der Lage sind, Holzreststoffe, wie z. B. Sägemehl zu verdauen. Auf die Fragen des Zusammenwirkens von Enzymen und Mikroorganismen soll im Rahmen dieser Anmeldung jedoch nicht eingegangen werden.

Die wichtigsten Eigenschaften einer Biogasanlage sind:
- Möglichst geringe Betriebskosten:
   Dies erfordert vor allem einen weitgehend störungsfreien Betrieb. Bei den hohen Investitionskosten für derartige Biogasanlagen müssen Anlagenstillstände für Inspektions- und Wartungsarbeiten, wobei zu letzteren auch solche insbesondere für die Reinigung derartiger Anlagen zu rechnen sind, möglichst vermieden werden.

Soweit Reinigungsarbeiten nicht zu umgehen sind, sollte der hierfür erforderliche Zeit- und Personalaufwand minimiert werden. Hier ist eine Anlagentechnik zu bevorzugen, die eine Reinigung verschmutzungsgefährdeter Bereiche, wie z. B. Mischer oder Wärmetauscher im eingebauten Zustand ermöglicht, möglichst ohne Betriebsunterbrechung. Hierfür wurde der Begriff der online-Reinigung geprägt.

Außerdem soll der Umsetzungsgrad der eingesetzten Biomasse möglichst hoch sein. Insbesondere sollen keine teuer zu entsorgenden Reststoffe anfallen. Ansonsten müsste nämlich z.B. eine Reststoffverbrennungsanlage oder, wenn der Schadstoffgehalt nicht zu groß ist, eine Kompostieranlage vorgesehen werden. Dies sind aber kostspielige Komponenten.
- Möglichst geringe Investitionskosten bezogen auf die produzierte Biogasmenge:
   Hier ist von entscheidender Bedeutung, dass "Reifungsprozesse" möglichst wenig Zeit benötigen, um die Behältergröße bei gegebenem Biogasoutput möglichst gering halten zu können. Die Biomasse sollte daher in einem zur Anwendung kommenden Verfahren optimal vorbereitet werden, und ferner sollte ein Besatz der zu behandelnden Biomasse mit Mikroorganismen möglichst angepasst sein. So ist es sicherlich nachteilig, wenn bei den bekanntermaßen sehr unterschiedlichen vorliegenden Zusammensetzungen von Biomasse der mikrobielle Besatz sich Im Wesentlichen auf gut verwertbare Anteile konzentriert, um diese umzusetzen, während der Rest der Biomasse kaum oder überhaupt nicht abgebaut wird.

Selbstverständlich sind Betriebs- und Investitionskosten hier eng miteinander verknüpft zu betrachten.

Mit der vorliegenden Erfindung wird das Ziel angestrebt, die vorstehend beschriebenen Eigenschaften einer Biogasanlage zu erreichen.

Die Steigerung des Durchsatzes der Biogasanlage wird einerseits durch ein spezielles Aufschlussverfahren in hierfür geeigneten Komponenten erreicht, insbesondere in einer mit hohen Strömungsgeschwindigkeiten betriebenen Mehrstoff- bzw. Mehrphasendüse.

In einer solchen Mehrstoff- oder Mehrphasendüse kann ein vorbereitetes Gemisch von Bioabfällen z.B. mit (Wasser)Dampf oder Druckluft behandelt werden. Die Strömungsgeschwindigkeiten des hierbei eingesetzten gasförmigen Fluids, also z. B. Dampf oder Druckluft können sowohl im hohen Unterschallbereich, im Bereich der Schallgeschwindigkeit oder sogar im Bereich der Überschallgeschwindigkeit liegen. Durch die von der Hochgeschwindigkeits-Strömung des gasförmigen Fluids auf das im Wesentlichen wässrige Gemisch aus organischem Material und einer Trägerflüssigkeit ausgeübten Kräfte, werden die Feststoffanteile des Gemisches zerkleinert, eingeschlossene Luftblasen, durch die die Oberfläche des' organischen Materials vor bakteriellem Besatz abgeschirmt werden kann, werden dabei freigesetzt und können anschließend über bekannte Entgasungsbauelemente ausgeschleust werden, je nachdem, ob eine aerobe oder anaerobe Vergärung nachfolgt. Dadurch wird das organische Material für die anschließende Verstoffwechselung unter Einwirkung von Mikroorganismen innerhalb der Biogasanlage bestens vorbereitet.

Für die Beschleunigung der Verstoffwechselung spielt bekanntermaßen auch die Temperatur eine erhebliche Rolle. Die Temperatur ist hierzu auf ein die Vermehrung der Keime und somit die Verstoffwechselung begünstigendes Niveau anzuheben. Es ist dabei bekannt, dass bei Temperaturen über 55 °C viele der hier relevanten Keimspezies abzusterben beginnen. Ein angestrebtes Optimum der Gasausbeute sollte demnach dann erzielt werden, wenn der mikrobielle Besatz gute Wachstumsbedingungen vorfindet. In für die entsprechende Temperaturanhebung üblicherweise benutzten Wärmetauschern, wie beispielsweise Röhren- oder Plattenwärmetauschern, besteht bei der hierfür durchzuführenden Beheizung einer Suspension aus organischem Material grundsätzlich das Risiko einer Belagsbildung und Inkrustierung an den Heizelementen.

Durch Einsatz z. B. von (Wasser-)Dampf oder Druckluft hoher Strömungsgeschwindigkeit in einer Mehrstoff- oder Mehrphasendüse wird ein entsprechendes Verschmutzungsrisiko erheblich reduziert.

Das Prinzip dieser Lösung ist insbesondere mit der Verwendung von Dampf bereits Stand der Technik. Bei besonders kritischen Materialien, wie z. B. bei grobpartikulären Speiseresten oder bei Strohhäcksel oder Maissilage kann es aber bei den bekannten herkömmlichen Verfahren, die mit Dampf in einer Mehrstoff- bzw. Mehrphasendüse arbeiten, zu Verstopfungen kommen.

Dies rührt daher, dass der Dampf bei den bekannten herkömmlichen Systemen üblicherweise zentral, d. h. innerhalb einer Düse radial innenliegend zugeführt wird, während die Feststoffsuspension über einen relativ schmalen radial außenliegenden Ringspalt, der um den zentralen Dampfzutritt herumläuft, in eine Mischkammer eingespeist wird. Bei einer Vorrichtung gemäß der vorliegenden Erfindung wird im Gegensatz dazu jedoch die Suspension radial innenliegend zentral mit einem im Wesentlichen geradlinig verlaufenden Rohr einer Mischkammer zugeführt.

Dadurch steht der feststoffbeladenen Suspension einerseits bei gleich großer Gesamtquerschnittsfläche ein größerer freier Strömungsquerschnitt zur Verfügung. Andererseits werden bei einer erfindungsgemäßen Ausführungsform, bei der die feststoffbeladene Suspension über ein Zentralrohr innerhalb einer Mischdüse geführt wird, von dieser keine Bauteile umströmt, an welchen sich längere Fasern, wie sie z. B. in Maissilage enthalten sind, ansammeln können.

Bei der Erfindung wird vorgeschlagen, eine Reinigungsvorrichtung durch das Zentralrohr bis zu der Mehrstoff- oder Mehrphasendüse, insbesondere bis zu deren Austrittsöffnung, oder durch diese hindurch bis in deren Mischkammer hinein oder auch darüber hinaus zu führen.

Die Reinigungsvorrichtung kann dabei z.B. auch über ein Ventil, über eine Dichtung oder über einen vergleichbaren Zugang in das Zentralrohr der Mehrstoff- oder Mehrphasendüse eingeführt werden. Dadurch ist es möglich, die Reinigungsvorrichtung nur bei Bedarf einer Reinigung in die Vorrichtung einzuführen. Im Normalbetrieb ist die Vorrichtung damit keiner Einschränkung unterworfen, die eine ständig eingesetzte Reinigungsvorrichtung bedeuten würde

Bei einer weiter bevorzugten Ausführungsform der Erfindung weist die Reinigungsvorrichtung einen Düsenkopf auf. Dieser ist beispielsweise an einer Zuleitung befestigt, über die eine Reinigungsflüssigkeit mit Hochdruck zu Reinigungszwecken in das Zentralrohr eingebracht werden kann.

Bei einer alternativen Ausführungsform weist die Reinigungsvorrichtung einen rotierbaren Fräskopf auf, der an einer rotierenden Antriebsstange befestigt ist. Über diese Antriebsstange kann einerseits die Rotation des Fräskopfes bewirkt werden und andererseits kann er auch in dem Zentralrohr bedarfsgerecht verschoben werden, um eine Reinigung dieser Leitung auf eine größere Länge zu erzielen.

Der angesprochene Düsenkopf oder der angesprochene Fräskopf mit ihren Zuleitungen bzw. Anstriebsstangen sind vorzugsweise so ausgebildet, dass die mit ihnen gebildete Reinigungsvorrichtung nur einen Teil des freien Querschnitts des Zentralrohrs beansprucht, sodass die Zuführung von Suspension aus organischem Material während eines Reinigungsprozesses nicht unterbrochen werden muss.

Das gasförmige Fluid, also (Wasser-)Dampf oder Druckluft, wird bei einer erfindungsgemäßen Vorrichtung über einen relativ schmalen Ringspalt mit sehr hoher Strömungsgeschwindigkeit in die Mischkammer eingeleitet. Der von dem Dampf oder von Druckluft durchströmte Querschnitt ist bei der üblichen Dampf- bzw. Druckluftqualität von industrieilen Dampf- oder Drucklufterzeugern nicht verschmutzungsgefährdet.

Von entscheidender Bedeutung ist es für die Leistungsfähigkeit einer Anlage, dass die zur Verstoffwechselung erforderlichen Mikroorganismen möglichst gleichmäßig auf die Biomasse verteilt werden und dass die Biomasse nicht durch Beläge gegen eine Verstoffwechselung abgeschirmt ist. Auch Lufteinschlüsse z. B. in hohlen Halmen können hier nachteilig sein. Daher kommt auch einer Entgasung bzw. der Ausleitung von Luft aus der Biomasse vor der eigentlichen Biogasproduktion eine erhebliche Bedeutung zu, jedenfalls bei anaeroben Vergasungsprozessen. Diese Entgasung kann durch die Behandlung in der Mehrstoff- oder Mehrphasendüse eingeleitet und In nachfolgenden Entgasem zum Abschluss gebracht werden.

Für die schnelle Umsetzung der Biomasse durch Einwirkung von Mikroorganismen muss das mikrobielle Inventar groß genug sein. Ferner müssen die Spezies des mikrobiellen Besatzes auf die umzusetzende Biomasse abgestimmt sein und möglichst gleichmäßig über die Biomasse verteilt werden.

Bei der Biogaserzeugung ist es bekannt, dass sie in mehreren Schritten erfolgt. In einem ersten Schritt, der Hydrolyse, werden hochmolekulare organische Stoffe (Kohlenhydrate, Fette, Eiweiße) mit Hilfe von Exo-Enzymen in niedermolekulare Bausteine (Dimere und Monomere) gespalten.

Bei diesem Schritt spricht man von Hydrolyse. Die Hydrolyse ist die Reaktion, die am langsamsten abläuft und somit die Geschwindigkeit des Abbauprozesses bestimmt.

Die Monomere und Dimere können nun in einem zweiten Schritt, der Fermentation, von Bakterien aufgenommen und abgebaut werden. Bei der Fermentation, auch Versäuerung genannt, werden hauptsächlich Carboxylsäuren (FOS) und Alkohole, aber auch in kleinen Mengen Kohlendioxid und Wasserstoff, gebildet.

In einem dritten Schritt bauen obligat Protonen reduzierende Bakterien die Carboxylsäuren (Butter- und Propionsäure) in Essigsäure, Wasserstoff und Kohlendioxid ab. Diese stark endotherme Reaktion kann nur ablaufen, wenn der Wasserstoffpartialdruck auf unter 10-5 bar sinkt.

Dies geschieht durch die eigentlichen Methanbakterien, die einen vierten und letzten Schritt ermöglichen. Obwohl das aus H₂ und CO₂ gebildete Methan nur 28 % ausmacht (der größere Teil wird aus Essigsäure gebildet), stellt diese Reaktion den Motor des Vergärungsprozesses dar.

Die Zusammensetzung der zu verarbeitenden Biomasse hinsichtlich der Bestandteile Kohlenhydrate, Eiweiße und Fette beeinflusst die Art der freiwerdenden Zwischenprodukte und die Geschwindigkeit der Hydrolyse. Große Bedeutung für den Gesamtprozess hat der Wasserstoffgehalt im System. Wird der Wasserstoff nicht in ausreichendem Maße abgeführt, kommt es zu ungünstigen thermodynamischen Verhältnissen und schwer abbaubare Zwischenprodukte werden gebildet.

Aus dem Zusammenspiel der verschiedenen Bakterien lässt sich erkennen, dass die Methanvergärung ein recht empfindlicher Prozess ist, anfällig für Störungen, wenn nicht alle chemischen Randbedingungen erfüllt sind.

Es wird davon ausgegangen, dass für die Methanbildung in Biogasanlagen auch Archaeen verantwortlich sind, die allerdings Stoffwechselprodukte von Pilzen benötigen, die sich ebenfalls im mikrobiellen Inventar befinden. Die Methanbildung ist unter mikrobiellen Gesichtspunkten somit als ein mehrstufiger, komplexer Prozess anzusehen.

Zur gleichmäßigen Verteilung der Mikroorganismen auf der Biomasse wird vorgeschlagen, Keimagglomerate, die sich insbesondere im Zuge einer längeren Bebrütungsphase an Partikeln bilden können, zu dispergieren und über die Biomasse zu verteilen.

Gemäß dem erfindungsgemäßen Verfahren ist vorgesehen, Mikroorganismen wie oben angesprochen, die für die Umsetzung der jeweiligen Biomasse optimal geeignet sind, in einem gesonderten Behälter sozusagen als Impfsuspension vorzubereiten.

Diese Impfsuspension kann in das umzusetzende Biomasse-Gemisch eingeleitet werden. Diese Einleitung in das Biomasse-Gemisch kann stromauf der Mehrstoff- und Mehrphasendüse erfolgen, sofern die Behandlung in dieser beaufschlagten Düse mit Dampf nicht zu einem Abtöten der Mikroorganismen führt.

Alternativ wird vorgeschlagen, die Impfsuspension getrennt und schonender zu dispergieren und dann ohne großen Stress für das mikrobielle Inventar in das Biomassegemisch einzuleiten.

Hierbei ist auch zu berücksichtigen, dass Dampf relativ teuer ist und zu einer Aufheizung der hiermit behandelten Suspension führt. Diese Aufheizung kann vorteilhaft sein, wenn die Temperatur der Suspension für eine schnelle Vermehrung der Mikroorganismen bzw. für die Verstoffwechselung noch zu niedrig ist.

Wenn die Suspension aber bereits erwärmt aus einer Vorbehandlungsstufe kommt, z. B. aus einer ersten Vergärungsstufe, könnte eine Behandlung mit Dampf zu einer Aufheizung führen, die oberhalb einer optimalen Prozesstemperatur liegt. In diesem Falle könnte zwar ein Wärmetauscher zur Abkühlung der Suspension eingesetzt werden. Derartige Wärmetauscher sind jedoch stark verschmutzungsgefährdet. Daher wird für solche Fälle gemäß einer alternativen Ausführungsform der Erfindung vorgeschlagen, Druckluft anstelle von Dampf in die Mehrstoff- bzw. Mehrphasendüse einzuleiten. Bei dieser Ausführungsform kann einer nachfolgenden Entgasung eine besondere Bedeutung zukommen, um ein Aufschäumen des Gemisches zu verhindern.

Nachfolgend wird eine Ausführungsform der Erfindung anhand einer Zeichnung beschrieben. Dabei zeigt:
- Figur 1: die Prinzipskizze einer Vorrichtung.
- Figur 2: die Prinzipskizze einer Mehrstoff- oder Mehrphasendüse zum Aufschluss von Biomasse unter Einsatz eines gasförmigen Fluids.

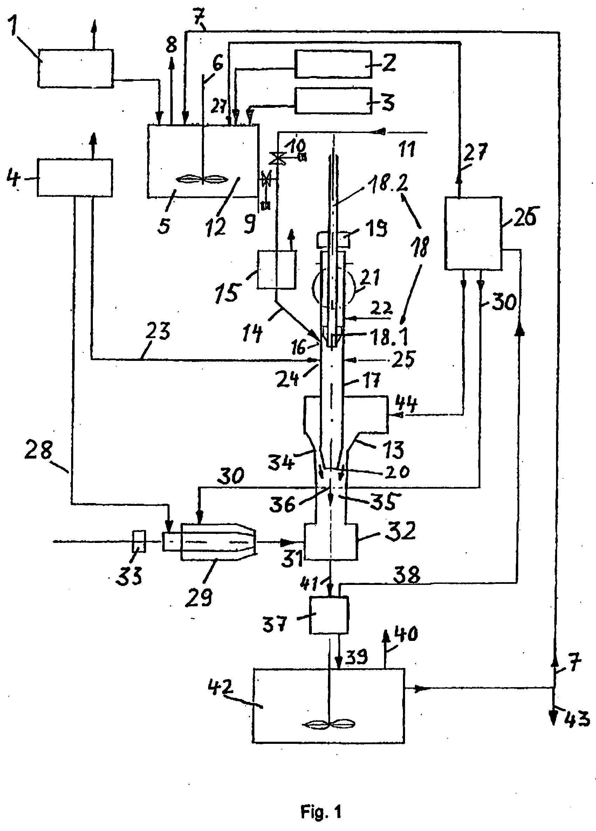

Fig. 1 zeigt im Wesentlichen jenen Teil einer Biogasanlage, in dem sich die Vorbehandlung der Biomasse abspielt, während die eigentliche Biogasproduktion hier nicht näher betrachtet bzw. beschrieben wird.

Zu behandelnde biologische Stoffe werden in Vorlagebehältern 1, 2 und 3 gespeichert bzw. vorgelegt. So kann beispielsweise in Behälter 1 Gülle, in Behälter 2 Strohhäcksel und in Behälter 3 Maissilage enthalten sein.

In einem separaten Behälter 4 ist bei der hier dargestellten Ausführungsform eine spezielle Suspension enthalten, die ausgesuchte Mikroorganismen und/oder Enzyme unter idealen Wachstumsbedingungen in einer Art Nährlösung enthält. Diese Suspension wird hier als Impfsuspension bezeichnet, denn mit dieser Suspension wird die vorbehandelte Mischung aus den Behältern 1, 2 und 3 geimpft, um einen optimalen mikrobiellen Umsatz zu erzielen.

Ausgehend von den Behältern 1, 2 und 3 wird ein Vorlagesammelbehälter 5 beschickt. Dieser ist mit einem Rührer 6 und mit einem Entgasungsstutzen 8 ausgestattet.

Der Entgasungsstutzen 8 ist vorgesehen, da ein erhöhter Gasgehalt in der Vorlage im Behälter 5 in der nachfolgenden Anlage zu übermäßiger Schaumbildung führen könnte.

Zur Vorwärmung des Gemisches 12 kann über eine Leitung 27 Dampf aus einem Dampferzeuger 26 In den Behälter 5 eingespeist werden.

Über ein Ventil 9 wird das Gemisch 12 aus dem Vorlagebehälter 5 einer Mehrstoff- bzw. Mehrphasendüse 13 zugeführt.

Die Verbindungsleitung 14 zwischen dem Ventil 9 und der Mehrstoff- bzw. Mehrphasendüse 9 kann über ein Ventil 10 mit Reinigungsflüssigkeit 11 beaufschlagt werden.

In die Verbindungsleitung 14 ist auch ein Entgaser 15 eingebaut. Dieser ist vor allem dann effizient, wenn es in der Verbindungsleitung 14 zu einer relevanten Druckabsenkung kommt, sodass zunächst gelöstes Gas (Luft) ausgasen und im Entgaser 15 aus der Strömung abgeschieden werden kann.

Die so vorbehandelte Suspension, die z. B. auch größere Strohhäckseistücke enthalten kann, wird bei der Mündung 16 seitlich in ein Zentralrohr 17 der Mehrstoff- bzw. Mehrphasendüse 13 eingespeist. Koaxial zu diesem Zentralrohr 17 ist dabei eine Reinigungsvorrichtung 18 eingebaut. Diese kann mit Hilfe eines Motors 19 in Rotation versetzt und axial bis zum Düsenmund 20 oder sogar darüber hinaus in koaxial angeordnete nachfolgende Komponenten verfahren werden.

Über ein Kugelventil 21 kann die Reinigungsvorrichtung 18 vollständig aus dem Zentralrohr herausgezogen werden.

Es ist hier zu erkennen, dass die Reinigungsvorrichtung 18 einen Düsenkopf 18.1 aufweist. Dieser ist an einer Zuleitung 18.2 befestigt, über die eine Reinigungsflüssigkeit mit Hochdruck zwischen 12 bar und 150 bar, vorzugsweise mit 50 bar, zu Reinigungszwecken in das Zentralrohr 17 eingebracht werden kann.

Bei einer alternativen Ausführungsform weist die Reinigungsvorrichtung 18 einen rotierbaren Fräskopf auf, der an einer rotierenden Antriebsstange befestigt ist. Über diese Antriebsstange kann einerseits die Rotation des Fräskopfes bewirkt werden und andererseits kann er auch in dem Zentralrohr bedarfsgerecht verschoben werden, um eine Reinigung dieser Leitung auf eine größere Länge zu erzielen. (Bei einer zeichnerischen Darstellung dieser alternativen Ausführungsform würde diese der Figur 1 entsprechen. Lediglich der dort dargestellte Düsenkopf 18.1 würde dem Fräskopf entsprechen und die Zuleitung 18.2 der Antriebsstange.)

Der angesprochene Düsenkopf 18.1 (oder der angesprochene Fräskopf) mit ihrer Zuleitung 18.2 (bzw. Anstriebsstange) sind so ausgebildet, dass die mit Ihnen gebildete Reinigungsvorrichtung 18 nur einen Teil des freien Querschnitts des Zentralrohrs 17 beansprucht, sodass die Zuführung von Suspension aus organischem Material während eines Reinigungsprozesses nicht unterbrochen werden muss.

Bei der Einleitstelle 22 besteht die Möglichkeit, eine Reinigungsflüssigkeit in das Zentralrohr der Düse einzuleiten, um mit dieser den Reinigungsprozess durch die Reinigungsvorrichtung 18 zu unterstützen.

Die Reinigungsvorrichtung 18 kann an ihrem vorderen Ende statt mit einem Fräskopf oder einem Hochdrucksprühkopf mit einer Bürste ausgestattet werden. Zur Unterstützung der Reinigung, aber auch zum Dispergleren des Gemisches 12, kann bei einer weiteren Anschlussstelle 25 auch Druckluft in das Zentralrohr 17 eingeleitet werden. Dies setzt jedoch voraus, dass es nicht zu einem unbeherrschbaren Aufschäumen des Gemisches kommt. Um dieses zu verhindern ist im hier dargestellten Ausführungsbeispiel der Düse 13 ein Entgaser 37 nachgeschaltet, dessen Abluft 38 beispielsweise als Verbrennungsluft für den Dampferzeuger 26 genutzt werden kann.

Über eine Leitung 23 kann aus dem Behälter 4 Impfsuspension bei der Impfstelle 24 in das Zentralrohr 17 eingeleitet werden. Dies ist jedoch nur dann sinnvoll, wenn der Stress für die in der Suspension befindlichen Mikroorgansimen innerhalb der strömungstechnisch nachgeschalteten Mehrstoff- bzw. Mehrphasendüse 13 nicht zu groß ist, sodass Mikroorganismen, die nachfolgend für die Verstoffwechselung benötigt werden, nicht in der Düse 13 abgetötet werden.

Falls dies zu befürchten ist, wird die Impfsuspension alternativ über eine Leitung 28 einer eigenen schonender arbeitenden Mischvorrichtung 29 zugeführt, in der Keimagglomerate aufgespalten werden können, ohne die Keime abzutöten. Auch diese Mischvorrichtung 29 kann über eine Leitung 30 als dampf- oder luftbeaufschlagte Mehrstoff- bzw. Mehrphasendüse betrieben werden, die evtl. auch mit einer eigenen online-Reinigungsvorrichtung 33 ausgestattet ist. In diesem Falle wird die Impfsuspension bei der Zuführstelle 31 In den Mischer 32 eingespeist.

Zur schonenden Dispergierung der Keimagglomerate könnte statt der beschriebenen dampf- oder luftbetriebenen Mischvorrichtung 29 allerdings auch ein niedrigenergetisch betriebenes Ultraschallsystem eingesetzt werden.

Das Gemisch 12 aus dem Vorlagebehälter 5 wird wie erwähnt über das Zentralrohr 17 der Mehrstoff- oder Mehrphasendüse 13 zugeführt.,

Dabei wird im vorliegend beschriebenen Fall Dampf über einen Ringspalt 34 dem Mischraum 35 der Düse 13 zugeführt, während das Gemisch 12 aus dem Vorlagebehälter 5 als Axialstrahl 36 in den Mischraum 35 einströmt. Es folgt der bereits beschriebene Mischer 32, in welchem die Impfsuspension dem aus der Düse 13 austretenden Gemisch zugeführt werden kann. Von hier aus strömt das Gemisch 41 in den weiteren Entgaser 37, der über eine Leitung 38 entlüftet wird.

Das derart von nicht brennbaren Gasen befreite Gemisch gelangt anschließend bei der Einfüllöffnung 39 in den Behälter 42 bzw. in einen hier nicht dargestellten ausgedehnten Anlagenteil, welcher der Biogasproduktion dient.

Es kann vorteilhaft sein, schwer zu verstoffwechselnde Komponenten aus diesem Behälter 42 über eine Leitung 7 in den Vorlagebehälter 5 zurückzuführen und einer zweiten Behandlung zu unterziehen. Alternativ können schwer zu verstoffwechselnde Komponenten über eine Leitung 43 ausgeschleust und z. B. einer thermischen Verwertung über eine Abfallverbrennungsanlage zur Dampferzeugung zugeführt werden.

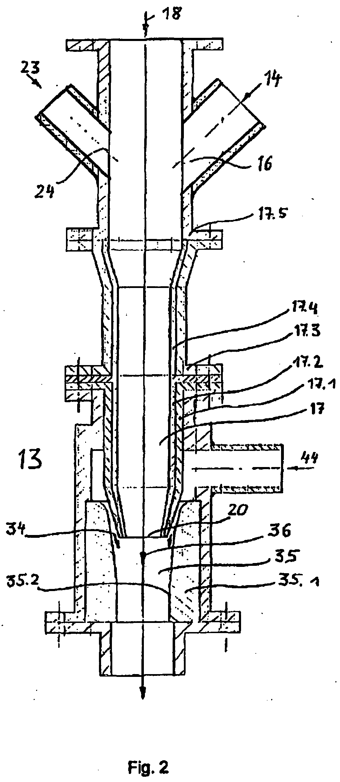

Fig. 2 zeigt die Düse 13 Im Detail. Die oben benutzten Bezugszeichen werden für die gleichen Hauptkomponenten generell beibehalten, wobei Einzelteile bzw. Oberflächenabschnitte dieser Hauptkomponenten innerhalb der Fig. 2 durch nachgestellte Ziffern gekennzeichnet sind.

Das Zentralrohr 17 ist im Bereich hoher Strömungsgeschwindigkeiten zum Schutz gegen Erosionsschäden mit Hartmetallbüchsen 17.2 und 17.4 ausgekleidet.

Zur Wärmedämmung ist das Zentralrohr im Abschnitt der Hartmetallbüchse 17.2 außerdem von einem Mantel 17.1 aus PTFE oder aus einem anderen geeigneten Material umgeben. Stromauf schließt sich der Zentralrohrabschnitt 17.5 an. Hier wird bei der Mündung 16 die Mischung 12 aus dem Vorlagebehälter 5 (siehe Fig. 1) seitlich eingespeist, sodass das Zentralrohr 17 für das Eindringen der axial arbeitenden Reinigungsvorrichtung 18 nicht durch starre Einbauten versperrt wird.

Dargestellt ist auch die Einspeisestelle 24 für die Impfsuspension 23. Diese Einspeisestelle wird jedoch nur dann gewählt, wenn die Behandlung des Gemisches 12 bzw. 14 in der Düse 13 nicht aufgrund zu hoher Temperaturen, die durch Dampf innerhalb der Düse 17 erzeugt werden, zu einem unvertretbar starken Absterben des mikrobiellen Inventars führt.

Die Mischkammer 35 der Düse 13 ist mit einem ringförmigen Körper 35.1 ausgekleidet, der eine einfache Anpassung des Ringspalts 34 an die jeweiligen Erfordernisse ermöglicht. Auch dieser Ringkörper ist aus Hartmetall oder aus Siliciumcarbid gefertigt, um Abrasionsschäden bei der Verarbeitung sandhaltiger Gemische in Grenzen zu halten.

Ferner ist die der Strömung 36 zugewandte Seite 35.2 des ringförmigen Körpers 35.1 mit hier nicht dargestellten Turbulenzgeneratoren versehen, die eine schnelle Vermischung wärmerer und kälterer Komponenten des Gemisches bewirken. Diese Turbulenzgeneratoren sind derart geformt, z. B. durch Abrunden von die Turbulenz erzeugenden Noppen, dass es nicht zu einem Herausfischen und Anhäufen langer Fasern an diesen Noppen kommen kann.

Abgesehen davon bietet die koaxiale Anordnung des Zentralrohres 17 in der Düse 13 und der nachfolgenden Mischkammer 35 die Möglichkeit, die Reinigungsvorrichtung 18 auch in die Mischkammer 35 hinein bzw. sogar darüber hinaus zu verfahren.

Zur Begriffsbildung sei auf folgenden Sachverhalt hingewiesen: In der Literatur ist es üblich, von einer Zweistoffdüse zu sprechen, wenn eine Flüssigkeit mit Hilfe eines Gases, in der Regel mit Druckluft, zerstäubt wird. Dabei entsteht ein mit Tropfen beladener Gasstrahl. Im vorliegenden Fall werden zwar auch eine flüssige und eine gasförmige Phase (zum Beispiel überhitzter Wasser-Dampf) miteinander in Kontakt gebracht, jedoch kondensiert in diesem Fall der Dampf sehr schnell vollständig an der flüssigen Phase, sodass eine flüssige Einphasenströmung zustande kommt. Im Sinne der Düsentechnik liegt dann keine Zweiphasendüse vor, sondern eine Flüssigkeitsdüse mit Erhitzung durch Dampfeinspeisung, wobei der Dampf kondensiert.

Eine solche Vorrichtung bzw. das Verfahren wären somit eher unter den Begriffen "Wärmetauscher" bzw. "mit Dampf betriebener Flüssigkeitserhitzer mit Dispergierungswirkung" einzuordnen.

## Patentansprüche

1. Mehrstoff- und Mehrphasendüse für die Behandlung von Biomasse für eine Biogasanlage, mit einem Zentralrohr, das mit einer Düsenmündung im Bereich eines Mischraumes endet, und mit einem Spalt, der die Mündung des Zentralrohres ringförmig umschließt,
**dadurch gekennzeichnet,**
**dass** sie eine Reinigungsvorrichtung (18) aufweist, die in das Zentralrohr (17) bis zur Düsenmündung (20) einführbar ist.

2. Mehrstoff- und Mehrphasendüse nach Anspruch 1,
**dadurch gekennzeichnet,**
**dass** die Reinigungsvorrichtung (18) einen Düsenkopf (18.1) aufweist, mit dem über eine Zuleitung (18.2) eine Reinigungsflüssigkeit mit Hochdruck in das Zentralrohr (17) eingebracht wird.

3. Mehrstoff- und Mehrphasendüse nach Anspruch 1,
**dadurch gekennzeichnet,**
**dass** die Reinigungsvorrichtung einen rotierbaren Fräskopf aufweist.

4. Mehrstoff- und Mehrphasendüse nach einem oder mehreren der Ansprüche 1 - 3,
**dadurch gekennzeichnet,**
**dass** die Reinigungsvorrichtung (18) nur einen Teil des freien Querschnitts des Zentralrohrs (17) versperrt, sodass die Zuführung von Gemisch (12) während eines Reinigungsprozesses nicht zu unterbrechen ist.

5. Mehrstoff- und Mehrphasendüse nach einem oder mehreren der Ansprüche 1 - 4,
**dadurch gekennzeichnet,**
**dass** die Mischkammer (35) einen sie umschließende Körper (35.1) aufweist, der mit Turbulenz erzeugenden Noppen ausgestattet ist.

6. Verfahren zur Behandlung von Bioabfällen in einer Biogasanlage, mit einem Vorlagebehälter (5) für die Vorbereitung eines Gemisches (12), sowie mit einer Mehrstoff- und Mehrphasendüse (13) gemäß einem oder mehreren der Ansprüche 1 bis 5 zur Behandlung des vorbereiteten Gemisches
**dadurch gekennzeichnet,**
**dass** eine Impfsuspension als mikrobieller Keimträger in einem gesonderten Behälter (4) zur Bebrütung und zur Vermehrung von Mikroorganismen zwischengespeichert wird und als Impfung in das Gemisch (12) eingespeist wird.

7. Verfahren zur Behandlung von Bioabfällen in einer Biogasanlage nach Anspruch 6,
**dadurch gekennzeichnet,**
**dass** die Impfsuspension erst hinter der Mehrstoff- und Mehrphasendüse (13) bzw. hinter einer dieser zuzuordnenden Mischkammer (35) in das Gemisch (12) eingespeist wird.

8. Verfahren zur Behandlung von Bioabfällen in einer Biogasanlage nach einem der Ansprüche 6 oder 7,
**dadurch gekennzeichnet,**
**dass** mikrobielle Keimagglomerate in der Impfsuspension vor dem Einmischen in das Gemisch in einem gesonderten Anlagenelement (29) dispergiert werden.

9. Verfahren zur Behandlung von Bioabfällen in einer Biogasanlage nach Anspruch 8,
**dadurch gekennzeichnet,**
**dass** zum schonenden Dispergieren der mikrobiellen Keimagglomerate in der Impfsuspension (4) eine Mehrstoff- und/oder Mehrphasendüse nach einem der Ansprüche 1 - 5 eingesetzt wird, wobei das schonende Dispergieren der mikrobiellen Keimagglomerate durch Absenken der Strömungsgeschwindigkeit bzw. der Temperatur auf vorteilhafte Werte bewirkt wird.

## Claims

1. A multicomponent and multiphase nozzle for the treatment of biomass for a biogas installation including a central tube, which terminates with a nozzle outlet in the vicinity of a mixing space, and a gap, which annularly surrounds the outlet of the central tube, **characterised in that** it includes a cleaning device (18), which may be introduced into the central tube (17) as far as the nozzle outlet (20).

2. A multicomponent and multiphase nozzle as claimed in Claim 1, **characterised in that** the cleaning device (18) includes a nozzle head (18.1), with which a cleaning liquid is introduced under high pressure into the central tube (17) via a feed pipe (18.2).

3. A multicomponent and multiphase nozzle as claimed in Claim 1, **characterised in that** the cleaning device includes a rotatable milling head.

4. A multicomponent and multiphase nozzle as claimed in one or more of Claims 1-3, **characterised in that** the cleaning device (18) obstructs only a proportion of the free cross-section of the central tube (17) so that the supply of mixture (12) during the cleaning process is not interrupted.

5. A multicomponent and multiphase nozzle as claimed in one or more of Claims 1-4, **characterised in that** the mixing chamber (35) includes a body (35.1) surrounding it, which is equipped with turbulence-producing protuberances.

6. A method of treating biological waste in a biogas installation including a collection container (5) for the preparation of a mixture (12) and a multicomponent and multiphase nozzle (13) as claimed in one or more of Claims 1 to 5 for treating the prepared mixture, **characterised in that** an inoculation suspension is temporarily stored in the form of a microbial germ carrier in a separate container (4) for the incubation and proliferation of microorganisms and is fed into the mixture (12) as an inoculation.

7. A method of treating biological waste in a biogas installation as claimed in Claim 6, **characterised in that** the inoculation suspension is fed into the mixture (12) primarily downstream of the multicomponent and multiphase nozzle (13) or downstream of a mixing chamber (35) associated with it.

8. A method of treating biological waste in a biogas installation as claimed in one of Claims 6 or 7, **characterised in that** microbial germ agglomerates are dispersed in the inoculation suspension before mixing into the mixture in a separate installation element.

9. A method of treating biological waste in a biogas installation as claimed in Claim 8, **characterised in that** for the purpose of gently dispersing the microbial germ agglomerates in the inoculation suspension a multicomponent and/or multiphase nozzle as claimed in one of Claims 1-5 is used, wherein the gentle dispersion of the microbial germ agglomerates is effected by reducing the flow velocity or the temperature to advantageous values.

## Revendications

1. Buse multi-substances et multi-phases pour le traitement de biomasse pour une installation de biogaz, comportant un tube central, qui se termine par une embouchure de buse dans la zone d'une chambre de mélange, et une fente qui entoure annulairement l'embouchure du tube central,
**caractérisée en ce**
**qu'**elle présente un dispositif de nettoyage (18) qui peut être introduit dans le tube central (17) jusqu'à l'embouchure de buse (20).

2. Buse multi-substances et multi-phases selon la revendication 1,
**caractérisée en ce**
**que** le dispositif de nettoyage (18) présente une tête de buse (18.1) avec laquelle un liquide de nettoyage est introduit à haute pression dans le tube central (17) par une conduite d'amenée (18.2).

3. Buse multi-substances et multi-phases selon la revendication 1,
**caractérisée en ce**
**que** le dispositif de nettoyage présente une tête de fraisage rotative.

4. Buse multi-substances et multi-phases selon l'une ou plusieurs des revendications 1 à 3,
**caractérisée en ce**
**que** le dispositif de nettoyage (18) obstrue seulement une partie de la section libre du tube central (17), de sorte qu'il n'est pas nécessaire d'interrompre l'amenée de mélange (12) pendant un processus de nettoyage.

5. Buse multi-substances et multi-phases selon l'une ou plusieurs des revendications 1 à 4,
**caractérisée en ce**
**que** la chambre de mélange (35) présente un corps (35.1) qui l'entoure, lequel est équipé de bossages qui produisent une turbulence.

6. Procédé de traitement de déchets biologiques dans une installation de biogaz, avec un réservoir collecteur (5) pour la préparation d'un mélange (12), ainsi qu'avec une buse multi-substances et multi-phases (13) selon l'une ou plusieurs des revendications 1 à 5 pour traiter le mélangé préparé
**caractérisé en ce**
**qu'**une suspension d'inoculation sous la forme d'un porteur de germes microbiens est stockée de manière intermédiaire dans un réservoir séparé (4) pour l'incubation et pour la multiplication de microorganismes et injectée en tant qu'inoculation dans le mélange (12).

7. Procédé de traitement de déchets biologiques dans une installation de biogaz selon la revendication 6,
**caractérisé en ce**
**que** la suspension d'inoculation est injectée dans le mélange (12) seulement derrière la buse multi-substances et multi-phases (13) ou derrière une chambre de mélange (35) à associer à celle-ci.

8. Procédé de traitement de déchets biologiques dans une installation de biogaz selon l'une des revendications 6 ou 7,
**caractérisé en ce**
**que** des agglomérats de germes microbiens sont dispersés dans la suspension d'inoculation dans un élément d'installation (29) séparé avant l'introduction dans le mélange.

9. Procédé de traitement de déchets biologiques dans une installation de biogaz selon la revendication 8,
**caractérisé en ce**
**qu'**une buse multi-substances et multi-phases selon l'une des revendications 1 à 5 est utilisée pour la dispersion en douceur des agglomérats de germes microbiens dans la suspension d'inoculation (4), la dispersion en douceur des agglomérats de germes microbiens étant produite par diminution de la vitesse d'écoulement et/ou de la température à des valeurs avantageuses.
